(19) Europäisches Patentamt
European Patent Office
Office européen des brevets

(11) **EP 4 759 294 A1**

(12) **EUROPEAN PATENT APPLICATION**

(43) Date of publication:
**17.06.2026 Bulletin 2026/25**

(21) Application number: **24307102.4**

(22) Date of filing: **13.12.2024**

(51) International Patent Classification (IPC):
***A61K 8/04*** *(2006.01)* ***A61K 8/11*** *(2006.01)*

(52) Cooperative Patent Classification (CPC):
**A61K 8/04; A61K 8/11;** A61K 2800/412;
A61K 2800/56

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Ecole Nationale Supérieure des Arts et
Industries
Textiles (ENSAIT)
59100 Roubaix (FR)**

• **Université de Lille
59800 Lille (FR)**

(72) Inventors:
• **SALAÜN, Fabien
59100 ROUBAIX (FR)**
• **RAULT, François
59430 SAINT-POL-SUR-MER (FR)**

(74) Representative: **Regimbeau
20, rue de Chazelles
75847 Paris Cedex 17 (FR)**

(54) **CORE-SHELL MICROPARTICLES COMPRISING A LIPOPHOBIC ACTIVE INGREDIENT,
DISPERSION COMPRISING SAME, AND USE THEREOF IN TEXTILE OR COSMETIC
APPLICATIONS**

(57)    The invention relates to microparticles comprising an oil core C and a shell S, wherein
• the oil core C comprises a lipophilic active ingredient;
• the shell S is a product of a sol-gel reaction of a mixture comprising a tetraalkoxysilane of formula $Si(OR_1)_4$, a trialkoxyalkylsilane of formula $R_2Si(OR_3)_3$ and a particulate surface agent with a carbon content ranging from 0.5 to 50 wt% relative to the total weight of particulate surface agent,
$R_1$ being independently a $(C_1\text{-}C_6)$alkyl, $R_2$ being a $(C_1\text{-}C_6)$alkyl, and $R_3$ being independently a $(C_1\text{-}C_6)$alkyl;
• the hydrodynamic diameter of the microparticles ranges from 0.5 to 100 $\mu m$ as measured by laser diffraction spectroscopy.

The invention further relates to a process for manufacturing the microparticles

## Description

### FIELD OF INVENTION

The present invention relates to core-shell microparticles encapsulating a lipophilic active ingredient. The invention further relates to a dispersion comprising the core-shell microparticles, and the use of the core-shell microparticles or the dispersion comprising same in cosmetic and textile applications.

### TECHNICAL BACKGROUND

To date, most commercial core-shell microparticles used in particular in textile applications are produced by *in situ* polymerization methods, generally requiring the use of cross-linking agents such as formaldehyde or glutaraldehyde. Such cross-linking agents raise issues for health and /or the environment. Also, when the core-shell microparticles are exposed to temperatures of 150°C or above, they tend to lose their transparency by yellowing.

Alternative core-shell microparticles have been developed using silica-based shells, obtained by a sol-gel process in a Pickering oil-in-water emulsion. Such silica-based core-shell microparticles were for instance described by Zhao etal. (Langmuir 2014, 30, 4253-4261) and by Butstraen et al. (Powder Technology 2015, 284, 237-244).

However, the process of Zhao *et al.* for preparing the core-shell microparticles requires complex reagents to obtain silica nanoparticles functionalized with silica prepolymers, said prepolymers gluing the particles at the oil/water interface, and polymerizing to form the shell under basic conditions.

As for the silica-based core-shell microparticles of Butstraen *et al.,* they require a combination of a particulate surface agent and a surfactant to sufficiently stabilize the emulsion to allow encapsulation. However, even then, only aggregates of interconnected silica capsules are obtained.

There is therefore a need for well-defined core-shell microparticles encapsulating lipophilic active ingredients, suitable in particular for textile and cosmetic applications, whose process of preparation is preferably free of non-environmentally friendly ingredients such as cross-linking agents, and is compatible with industrial constraints, such as reproducibility, cost-effectiveness and ease of implementation. Also, the process should preferably not require high temperature, thus allowing the use of thermosensitive lipophilic active ingredients, and use easily available reagents. The obtained core-shell microparticles are advantageously heat-resistant, biocompatible and transparent, and suitable for contact with human skin.

### SUMMMARY OF INVENTION

The inventors have discovered that subjecting a mixture comprising a tetraalkoxysilane and a trialkoxyalkylsilane in the oil phase of a Pickering emulsion to a sol-gel reaction where the particulate surface agent has a carbon content of 0.5 wt% or more, relative to the total weight of particulate surface agent, allows to obtain microparticles solving the above technical problems. The combination of the use of a trialkoxyalkylsilane as gel precursor and of a particulate surface agent with a specific carbon content is of particular importance to obtain well-defined core-shell particles, with high loading efficiency in high yields.

Without wishing to be bound by theory, it is believed that the carbon content of the particulate surface agent favors the migration of the trialkoxyalkylsilanes (either in their monomeric form or in their hydrolyzed form) at the interface, favoring the formation of a shell around the oil phase droplets, rather than inside the oil phase.

Specifically, the invention relates to a process for preparing microparticles comprising an oil core C and a shell S, wherein

- the oil core C comprises a lipophilic active ingredient;
- the shell S is a product of a sol-gel reaction of a mixture comprising a tetraalkoxysilane of formula $Si(OR_1)_4$, a trialkoxyalkylsilane of formula $R_2Si(OR_3)_3$ and a particulate surface agent with a carbon content of 0.5 wt% or more, relative to the total weight of particulate surface agent,
  $R_1$ being independently a $(C_1-C_6)$alkyl, $R_2$ being a $(C_1-C_6)$alkyl, and $R_3$ being independently a $(C_1-C_6)$alkyl;
- the hydrodynamic diameter of the microparticles ranges from 0.5 to 100 $\mu$m as measured by laser diffraction spectroscopy.

the process, comprising the following steps:

a) Suspending a particulate surface agent with a carbon content of 0.5 wt% or more, relative to the total weight of particulate surface agent, in an aqueous phase Aq, preferably having a pH ranging from 3 to 10, to obtain an aqueous suspension,

b) Mixing the aqueous suspension with an oil phase comprising a lipophilic active ingredient, a tetraalkoxysilane of formula $Si(OR_1)_4$, and a trialkoxyalkylsilane of formula $R_2Si(OR_3)_3$, with $R_1$, $R_2$ and $R_3$ as defined above, to obtain a biphasic mixture,

c) Emulsifying the biphasic mixture to obtain an oil-in-water emulsion,

d) Subjecting the oil-in-water emulsion to gelling conditions, so as to obtain a dispersion of microparticles comprising the oil core C encapsulated into a shell S which is the product of a sol-gel reaction of a mixture comprising the tetraalkoxysilane, the trialkoxyalkylsilane and the particulate surface agent.

The invention further concerns microparticles comprising an oil core C and a shell S, wherein

- the oil core C comprises a lipophilic active ingredient;
- the shell S is a product of a sol-gel reaction of a mixture comprising a tetraalkoxysilane of formula $Si(OR_1)_4$, a trialkoxyalkylsilane of formula $R_2Si(OR_3)_3$ and a particulate surface agent with a carbon content of 0.5 wt% or more, relative to the total weight of particulate surface agent,
  $R_1$ being independently a $(C_1\text{-}C_6)$alkyl, $R_2$ being a $(C_1\text{-}C_6)$alkyl, and $R_3$ being independently a $(C_1\text{-}C_6)$alkyl;
- the hydrodynamic diameter of the microparticles ranges from 0.5 to 100 $\mu$m as measured by laser diffraction spectroscopy.

The microparticles are typically obtainable or obtained by the process of the invention.

The invention further relates to a dispersion comprising an aqueous phase and the microparticles of the invention or obtained by the process of the invention.

The invention further relates to a use of the microparticles of the invention or obtained by the process of the invention, or the dispersion of the invention, in a cosmetic composition, or to coat a surface, such as a textile surface.

## DETAILED DESCRIPTION

Any range of values denoted by the expression "between a and b" represents the range of values from greater than a to less than b (i.e., excluding bounds a and b) while any range of values denoted by the phrase "from a to b" means the range of values from a to b (i.e., including the strict bounds a and b).

As used herein, the terms "a" or "an" means "one or more than one".

As used herein, a composition "substantially free of" a component means that the composition comprises 0.01 wt% or less, preferably 0.001 wt% or less, of the component relative to the total weight of the composition.

As used herein, a composition "consists essentially of" or "consisting essentially of" a component means that the composition comprises at least 99.5 wt%, preferably at least 99.9 wt% of the component, relative to the total weight of the composition.

As used herein, "microparticles" are understood as solid particles typically of micrometric size with a mean diameter ranging from 0.5 $\mu$m to 100 $\mu$m.

As used herein, "essentially spherical" particles include spherical and/or nearly-spherical particles. In some embodiments, the substantially spherical metal powder can have an average particle aspect ratio of about 1.5 or less. In further embodiments, the average particle aspect ratio can range from about 0.9 to about 1.1. As used herein, "aspect ratio" refers to the longest dimension of a particle divided by the shortest dimension of the particle.

A "surface agent" is an agent able to modify the surface tension of an emulsion, particularly at the interface between the oil phase and the aqueous phase.

As used herein a « dispersion » refers to a heterogenous mixture of two immiscible phases, wherein which the dispersing or continuous phase is consists of a unique and vast domain and is generally liquid, while the dispersed or discontinuous phase consists of small separate domains also called droplets or particles and is generally solid or liquid. The suspension is advantageously colloidal, so that the dispersed phase does not or only scarcely sediments in the dispersing phase.

As used herein a « suspension » refers to a mixture in which the dispersing (or continuous) phase is liquid and the dispersed phase is solid (dispersion with a solid dispersed phase).

An emulsion is a dispersion with a liquid dispersed phase. As used herein an « oil-in-water emulsion » refers to a mixture in which the continuous phase is the aqueous phase while the dispersed phase is the liquid oil phase. An oil-in-water comprises an interface between the oil phase and the aqueous phase. The oil-in-water emulsion is a Pickering emulsion, thus with a particulate surface agent at the interface.

As used herein, the term "$(C_1\text{-}C_6)$alkyl"

As used herein, the term "$(C_1\text{-}C_6)$alkoxy" refers to a $(C_1\text{-}C_6)$alkyl group as defined above bound to the molecule via an oxygen atom, including, but not limited to, methoxy, ethoxy, n-propoxy, iso-propoxy, n-butoxy, iso-butoxy, sec-butoxy, t-butoxy, n-pentoxy, n-hexoxy, and the like.

## 1. Process for preparing the microparticles

The invention concerns a process for preparing microparticles, the process comprising the following steps:

a) Suspending a particulate surface agent with a carbon content of 0.5 wt% or more, relative to the total weight of particulate surface agent, in an aqueous phase Aq, preferably having a pH ranging from 3 to 10, to obtain an aqueous suspension,

b) Mixing the aqueous suspension with an oil phase comprising a lipophilic active ingredient, a tetraalkoxysilane of formula $Si(OR_1)_4$, and a trialkoxyalkylsilane of formula $R_2Si(OR_3)_3$, with $R_1$, $R_2$ and $R_3$ as defined above or below, to obtain a biphasic mixture,

c) Emulsifying the biphasic mixture to obtain an oil-in-water emulsion,

d) Subjecting the oil-in-water emulsion to gelling conditions, so as to obtain a dispersion of microparticles comprising the oil core C encapsulated into a shell S which is the product of a sol-gel reaction of a mixture comprising the tetraalkoxysilane, the trialkoxyalkylsilane and the particulate surface agent.

*Step a)*

The aqueous phase Aq of step a) comprises an aqueous solvent typically comprising water and optionally another solvent miscible with water, such as an alcohol.

As used herein, an "alcoholic solvent" or an "alcohol" is understood as a hydrocarbon in which a hydrogen atom has been replaced with an OH group. The hydrocarbon and may be linear or branched, saturated or unsaturated (i.e. containing one or several double bond C=C), preferably linear or branched and saturated. The alcohol is advantageously liquid at the temperature at which the method is performed, and/or at room temperature (*i.e.* from 15°C to 40°C, preferably from 20°C to 30°C). The alcoholic solvent is advantageously a lower alcohol, comprising advantageously 1 to 6 carbon atoms, notably 1 to 6 carbon atoms, such as 1 to 4 carbon atoms. It may be in particular 4 to 6 carbon atoms. It can be in particular methanol, ethanol, propanol, isopropanol, n-butanol and n-hexanol, advantageously methanol or ethanol. In some embodiments, the aqueous solvent consists of or essentially consists of water. In other words, in those embodiments, the aqueous phase Aq comprises water and no other solvent.

Advantageously, the aqueous phase Aq of step a) is acidic. Preferably, the aqueous phase has a pH ranging from 3 to 6, more preferably from 3 to 5.

The pH of the aqueous phase Aq may be adjusted by adding an acid, such as an organic or inorganic acid. In other words, the aqueous phase Aq comprises an organic or inorganic acid. Examples of inorganic acids are hydrochloric acid, hydrobromic acid, sulfuric acid, nitric acid, phosphoric acid and the like. Exemplary organic acids are acetic acid, benzenesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, mandelic acid, methanesulfonic acid, muconic acid, 2-naphtalenesulfonic acid, propionic acid, succinic acid, dibenzoyl-L-tartaric acid, tartaric acid, p-toluenesulfonic acid, trimethylacetic acid, trifluoroacetic acid and the like.

Preferably, the acid used to adjust the pH of the aqueous phase Aq is an organic acid, containing at least one carboxylic acid -COOH group. Advantageously, the organic acid is an acid of formula $(C_1-C_6)$alkyl-COOH or $(C_1-C_4)$alkyl-COOH, and even more preferably is acetic acid.

As used herein, a "particulate surface agent" refers to a surface agent in the form of solid particles, as opposed to surfactants. Surfactants are distinguished from particulate surface agents in that they do not include particles, be them solid or semi-solid.

Typically, the particulate surface agent does not vectorize any active ingredient: the particles of the particulate surface agent are thus typically free of active ingredient.

Advantageously, the particulate surface agent consists of nanoparticles, that is to say the particulate surface agent has an average particle size ranging from 1 to 100 nm, preferably from 2 to 50 nm, even more preferably from 5 to 20 nm, as measured by laser diffraction spectroscopy.

The particulate surface agent typically has a carbon content ranging from 0.5 to 50 wt%, preferably from 0.7 to 35 wt%, even more preferably from 0.8 to 10 wt%, relative to the total weight of the particulate surface agent. In some variants, the carbon content of the particulate surface agent ranges from 0.8 to 5 wt%, relative to the total weight of the particulate surface agent. In some variants, the carbon content of the particulate surface agent ranges from 12 to 40 wt%, relative to the total weight of the particulate surface agent.

The particulate surface agent may be selected from the group consisting of:

-    a silica carbide or a metal carbide such as aluminum carbide.
-    a hydrophobized silica;
-    a hydrophobized mixture of silica with a metal oxide, such as titanium oxide, manganese oxide, calcium oxide, calcium carbonate, zirconium oxide, or mixtures thereof;

- or mixtures thereof.

In some embodiments, the particulate surface agent is a hydrophobized silica, in particular a silica hydrophobized with a hydrophobing agent selected from the group consisting of organosiloxanes, organochlorosilanes and organoalkoxysilanes, advantageously the hydrophobing agent is selected from the group consisting of hexamethyldisiloxane (HMDSO), trimethylchlorosilane, trimethylethoxysilane, hexamethyldisilazane and hexadecylsilane, preferably it is hexadecylsilane.

The particulate surface agent is suspended in the aqueous phase Aq according to procedures known in the art.

In some embodiments, the particulate surface agent is suspended in the aqueous phase Aq and the pH of the aqueous suspension is then adjusted to a value ranging from 3 to 10, preferably from 3 to 6.

In some embodiments, the aqueous phase Aq already has a pH ranging from 3 to 10, preferably from 3 to 6, and there is no additional adjustement step after the suspension step a).

_Step b)_

In some embodiments, the aqueous suspension of step a) is added, preferably dropwise, to the oil phase. Conversely, in some embodiments, the oil phase is added, preferably dropwise, to the aqueous suspension of step a).

The oil phase comprises a lipophilic active ingredient.

As used herein, a "lipophilic active ingredient" is an active ingredient (or an ingredient of interest) with a low water solubility according to the Biopharmaceutics Classification System, as defined in particular in document « M9 Biopharmaceutics Classification System Based Biowaivers - Guidance for Industry » published in May 2019 by the American FDA (Food and Drug Administration), or document « ich-m9-biopharmaceutics-classification-system-based-biowaivers-step-5 » published by the EAM (European medicine agency) on 30/07/2020, and available online at the following address : https://www.ema.europa.eu/documents/scientific-guideline/ich-m9-biopharmaceutics-classification-system-based-bio waivers-step-5_en.pdf.

A "lipophilic active ingredient" is typically a BCS class II or IV active ingredients. Thus, an active ingredient is considered liposoluble when less than 85% by weight of the highest therapeutic dose is soluble in 250 mL of an aqueous solution buffered over a pH range from 1 to 6.8 at $37°C \pm 1°C$.

Advantageously, the boiling point of the lipophilic active ingredient is of 70°C or more, more preferably of 80°C or more.

In some embodiments, the lipophilic active ingredient has a melting point of 15°C or less, more preferably of 10°C or less, even more preferably of 5°C or less.

Advantageously, the lipophilic active ingredient is stable when in contact with an aqueous solution at a pH ranging from 3 to 5, more preferably from 3 to 6.

Advantageously, the lipophilic active ingredient is selected from the group consisting of:

- Pest control agent, for instance paraffins, in particular $(C_{12}-C_{20})$-hydrocarbons such as $(C_{16}-C_{20})$-_n_-alcane, in particular _n_-hexadecane, _n_-octadecane, n-eicosane;
- Essential oils;
- Perfumes;
- A vegetable oil such as an oil selected from the group consisting of neem oil, almond oil, sunflower oil, or mixtures thereof;
- Disinfectants and/or bactericides;
- Solar filters and/or UV filters, such as octylmethylcinnamate (OMC) for instance;
- Fire retardants, such as bisphenol diphenyl phosphate, resorcinol diphenyl phosphate;
- Soothing agents, such as cannabidiol;
- and mixtures thereof.

As used herein, a "$(C_{12}-C_{20})$ hydrocarbon" refers to a saturated linear or branched hydrocarbon, comprising from 12 to 20 carbon atoms, notably from 16 to 20 carbon atoms.

As used herein, a "$(C_{12}-C_{20})$ _n_-alcane" refers to a linear $(C_{12}-C_{20})$ hydrocarbon.

As used herein, an "essential oil" is understood as a concentrated hydrophobic liquid containing volatile chemical compounds from plants, generally containing the essence of the plant's fragrance. Essential oils are typically prepared by distilling, often by using steam, parts of the plant. Other processes to prepare essential oils include expression, solvent extraction, sfumatura, absolute oil extraction, resin tapping, of parts of the plant, wax embedding, and cold pressing. Essential oils are generally used as fragrances (for instance in perfumes, incense, air freshener), as cosmetic ingredients, as disinfectants, for flavoring food and drinks, as insect repulsive, as relaxing agent...

As used herein, "perfumes" are scented formulations, generally containing more than one ingredient. Perfumes may contain one or more essential oil, but are distinguished therefrom. As used herein, a "vegetable oil" is understood as an oil extracted from seeds or from other parts of edible plants by mechanical extraction, usually pressing. Vegetable oils are distinguished from essential oils in that they are not concentrated - as they are not obtained via a chemical process but by a purely mechanical process. Vegetable oils generally contain mixtures of triglycerides. Soybean oil, grape seed oil, and cocoa butter are examples of seed oils. Olive oil, palm oil, and rice bran oil are examples of fats from other parts of plants. Vegetable oils are typically liquid at room temperature, i.e. at 20°C or more, preferably at 15°C or more.

Neem oil is a vegetable oil generally obtained from Neem *(Azadirachta indica)* seeds, useful as a pest control, in particular as an insecticide.

Sweet almond oil is useful as cosmetic ingredient.

As used herein, a "soothing agent" is understood as a substance or a composition reducing, alleviating or preventing the sensation of anxiety, stress or nervousness. Examples of soothing agents include cannabidiol.

The oil phase may further comprise an oil, said oil being different from the lipophilic active ingredient. The use of an additional oil is preferred when the melting point of the lipophilic active ingredient is of 15°C or more: in such case, the additional oil is adapted to yield a liquid oil phase at a temperature ranging from 15°C to 25°C. The person of skill in the art will know how to select the oil and the concentration of the lipophilic ingredient in the oil to obtain a liquid oil phase at a temperature ranging from 15°C to 25°C. Therefore, the active ingredient may or may not be solubilized in an oil.

Advantageously, the oil is liquid at room temperature, that is to say the oil has a melting point of 20°C or less, preferably of 15°C or less. Preferably, the boiling point of the lipophilic active ingredient is of 70°C or more, more preferably of 80°C or more.

Advantageously, the oil is a vegetable oil as defined above (or another vegetable oil if the lipophilic active ingredient is also a vegetable oil).

In variants where the oil phase further comprises an oil, the concentration of the lipophilic active ingredient in the oil phase in step b) preferably ranges from 10% to 40% v/v.

The tetraalkoxysilane is typically of formula $Si(OR_1)_4$ with $R_1$ being independently a $(C_1-C_6)$alkyl. Examples of tetraalkoxysilanes include tetraethoxysilane (TEOS), tetramethoxysilane (TMOS), tetrapropoxysilane, tetrabutyl orthosilicate (or tetera-*n*-butyloxysilane). Preferably, the tetraalkoxysilane is TEOS.

The trialkoxyalkylsilane is typically of formula $R_2Si(OR_3)_3$, with $R_2$ being a $(C_1-C_6)$alkyl and $R_3$ being independently a $(C_1-C_6)$alkyl. Examples of include methyltriethoxysilane (MTES), ethyltriethoxysilane, ethyltrimethoxysilane, methyltrimethoxysilane, n-propyltriethoxysilane, methyltri-n-propoxysilane, ethyltri-n-propoxysilane. Advantageously, the trialkoxyalkylsilane is MTES.

Preferably, the tetraalkoxysilane is TEOS and the trialkoxyalkylsilane is MTES.

Preferably, the trialkoxyalkylsilane/tetraalkoxysilane mass ratio in step b) ranges from 30/70 to 70/30, preferably from 40/60 to 60/40, even more preferably from 45/55 to 55/45. Advantageously, the trialkoxyalkylsilane/tetraalkoxysilane mass ratio in step b) ranges from 50/50 to 70/30, preferably from 50/50 to 60/40, even more preferably from 50/50 to 55/45.

The particulate surface agent/oil phase mass ratio in step b) may range from 0.005 to 0.05, preferably from 0.005 to 0.015.

In some embodiments, the oil phase consists of the lipophilic active ingredient, the trialkoxyalkylsilane, the tetraalkoxysilane, and optionally the oil. In other words, the oil phase may comprise oil and no other solvent.

In some embodiments, the aqueous phase consists of water, the acid and the particulate surface agent, while the oil phase consists of the lipophilic active ingredient, the trialkoxyalkylsilane, the tetraalkoxysilane, and optionally the oil.

Typically, the dry weight of the biphasic mixture ranges from 5% to 50%, preferably from 10% to 40% (m/m or w/w).

In some embodiment, the mixing is performed at a temperature above melting point of the lipophilic active ingredient, preferably from 5°C to 10°C, provided that the temperature in is the range from 15°C to 80°C, so as to avoid or limit evaporation of the aqueous phase. Advantageously, the mixing is performed at a temperature ranging from 15°C to 40°C, such as from 15°C to 28°C.

*Emulsifying step c)*

The emulsifying step yields a Pickering emulsion, *i.e.* an emulsion wherein the particulate surface agent is at the interface between the aqueous phase and the oil phase and stabilizes the emulsion.

The emulsifying is performed by stirring, typically using a dispersing system such as a dispersing generator or a high shear dispersing machine.

The stirring speed of the dispersing system ranges from 5000 rpm to 15000 rpm (rotations per minute), such as from 7000 rpm to 12000 rpm. Typically, the rotating speed is 8000 rpm.

In some embodiments, the mixing step and the emulsifying step are concomitant.

Advantageously, the oil-in-water emulsion obtained in step c) comprises oil droplets having a number average diameter in number ranging from 0.5 $\mu$m to 50 $\mu$m, preferably from 5 $\mu$m to 20 $\mu$m.

In some embodiment, the emulsifying step is performed at a temperature above melting point of the lipophilic active ingredient, preferably from 5°C to 10°C, provided that the temperature in is the range from 15°C to 80°C. Advantageously, the emulsifying is performed at a temperature ranging from 15°C to 40°C, such as from 15°C to 28°C.

*Gelling step d)*

A sol-gel reaction produces a gel-like phase from a solution of gel precursors (a colloidal solution), called a sol.

Here, the gel precursors consist of the mixture of tetraalkoxysilane, trialkoxyalkylsilane and particulate surface agent. The sol - partly solubilized in the oil phase - slowly forms a gel at the interface of the Pickering emulsion, which will form the shell of the microparticles.

The sol-gel reaction comprises a hydrolysis step, followed by a condensation step.

The hydrolysis step yields silanols and alcohols, in particular according to equations (Eq. 1) and (Eq. 2):

$$Si(OR_1)_4 + H_2O \rightarrow (R_1O)_3\text{-Si-(OH)} + R_1\text{-OH} \qquad \text{(Eq. 1)}$$

$$R_2Si(OR_3)_3 + H_2O \rightarrow R_2(R_3O)_2\text{-Si-(OH)} + R_3\text{-OH} \qquad \text{(Eq. 2)}$$

The hydrolysis step is catalyzed under acidic conditions.

The condensation step in turn comprises 2 sub-steps: a polymerization step and a maturation step.

During the polymerization hydrolyzed silanols react on each other to form polysiloxanes containing at least one Si-O-Si bond, by:

- oxolation, wherein 2 hydroxyls groups from silanols react with each other by nucleophilic substitution (see e.g. equations (Eq. 3), (Eq. 4) and (Eq. 5)):

$$2\ (R_1O)_3\text{-Si-(OH)} \rightarrow (R_1O)_3\text{-Si-O-Si-}(OR_1)_3 + H_2O \qquad \text{(Eq. 3)}$$

$$2\ R_2(R_3O)_2\text{-Si-(OH)} \rightarrow R_2(R_3O)_2\text{-Si-O-Si-}(OR_3)_2R_2 + H_2O \qquad \text{(Eq. 4)}$$

$$(R_1O)_3\text{-Si-(OH)} + R_2(R_3O)_2\text{-Si-(OH)} \rightarrow R_2(R_3O)_2\text{-Si-O-Si-}(OR_1)_3 + H_2O \qquad \text{(Eq. 5)}$$

- and/or alcoxolation, wherein a reaction occurs between an alcohol group and a -OH group, (see e.g. equations (Eq. 6) to (Eq. 9)):

$$(R_1O)_3\text{-Si-(OH)} + Si(OR_1)_4 \rightarrow (R_1O)_3\text{-Si-O-Si-}(OR_1)_3 + R_1OH \qquad \text{(Eq. 6)}$$

$$R_2(R_3O)_2\text{-Si-(OH)} + Si(OR_1)_4 \rightarrow R_2(R_30)_2\text{-Si-O-Si-}(OR_1)_3 + RiOH \qquad \text{(Eq. 7)}$$

$$(R_1O)_3\text{-Si-(OH)} + R_2Si(OR_3)_3 \rightarrow R_2(R_3O)_2\text{-Si-O-Si-}(OR_1)_3 + R_3OH \qquad \text{(Eq. 8)}$$

$$R_2(R_3O)_2\text{-Si-(OH)} + R_2Si(OR_3)_3 \rightarrow R_2(R_3O)_2\text{-Si-O-Si-}(OR_3)_2R_2 + R_3OH \qquad \text{(Eq. 9)}$$

Of course, during polymerization, the oxolation and alcoxolation reactions also occur on polysiloxanes molecules (*i.e.* already polymerized silanols, to yied polysiloxanes containing more than one Si-O-Si bond) to form longer polysiloxanes polymeric chains.

The maturation step comprises further cross-linking of the polysiloxanes polymeric chains, thus reinforcing the mechanical strength of the shell.

It should be noted that the silanols also react with the particulate surface agent during polymerization and maturation. Indeed, the particulate surface agent also contains reaction Si(OH) bonds or M-(OH) bonds (wherein M is a metal).

The shell under formation thus includes surface agent particles covalently bonded to the polysiloxanes polymeric chains.

The gelling step includes both the hydrolysis and the condensation step of the sol-gel reaction. In some embodiments, the oil-in-water emulsion has an aqueous phase having a pH ranging from 3 to 10, preferably 3 to 6, and the step d) of subjecting the emulsion to gelling conditions comprises the following sub-steps:

d1) Adding a base to the oil-in-water emulsion so as to raise the pH of the aqueous phase of the emulsion to a range from 5 to 11, preferably from 9 to 10, thereby obtaining an emulsion with an adjusted pH,

d2) Heating the emulsion with an adjusted pH to a temperature below the lowest ebullition temperature between the ebullition temperature of the lipophilic active ingredient, water, the trialkoxyalkylsilane and the tetraalkoxysilane, preferably ranging from 45°C to 80°C such as from 50°C to 70°C, to obtain a hot dispersion,

d3) Cooling the hot dispersion to room temperature.

The pH of the aqueous phase of the basified emulsion obtained in step d1) is greater than the pH of the aqueous phase of the emulsion obtained in step c).

The base may be organic or inorganic. Examples of organic base are diethanolamine, ethanolamine, N-methylglucamine, triethanolamine, tromethamine and the like. Examples or inorganic base are aluminium hydroxide, calcium hydroxide, potassium hydroxide, sodium carbonate and ammonium hydroxide. Preferably, the base is an inorganic base such as ammonium hydroxide.

Preferably, in step d2), the temperature is from 10°C to 20°C above the melting point of the lipophilic active ingredient, provided that the temperature is in the range from 45°C to 80°C, preferably 50°C to 70°C.

Advantageously, steps d1) and d2) are performed under stirring, at a stirring speed of 300 rpm to 800 rpm, such as from 400 rpm to 700 rpm.

*Optional steps*

In some embodiments, the process further comprises a subsequent step e) of isolating the microparticles, for instance by filtration, atomization or centrifugation, and optionally drying the microparticles. Preferably, the step e) comprises or consists of isolating the microparticles by filtration, and optionally drying the microparticles.

The filtration may be performed using any technique known in the art. In a variant, the filtration is carried out under reduced pressure.

Drying techniques are known in the art. Preferably, drying is performed by placing the particles in an oven at a temperature ranging from 25°C to 80°C, for a predetermined time. Advantageously, the oven is ventilated, to accelerate drying. Alternatively, the drying may be performed using filter paper.

*Microparticles obtained by the process*

The process yields microparticles comprising an oil core C and a shell S, wherein

- the oil core C comprises a lipophilic active ingredient;
- the shell S is the product of a sol-gel reaction of a mixture comprising a tetraalkoxysilane of formula $Si(OR_1)_4$ , a trialkoxyalkylsilane of formula $R_2Si(OR_3)_3$ and the particulate surface agent, with $R_1$, $R_2$, $R_3$ as defined herein;
- the hydrodynamic diameter of the microparticles ranges from 0.5 to 100 $\mu$m as measured by laser diffraction spectroscopy.

Advantageously, the obtained microparticles are substantially free of cross-linking agent, and/or substantially free of surfactant. In other words, advantageously, no cross-linking agent or surfactant is used in the process.

Typically, microparticles are essentially spherical.

## 2. **Microparticles**

The present invention concerns microparticles comprising an oil core C and a shell S, wherein

- the oil core C comprises a lipophilic active ingredient;
- the shell S is the product of a sol-gel reaction of a mixture comprising a tetraalkoxysilane of formula $Si(OR_1)_4$ , a trialkoxyalkylsilane of formula $R_2Si(OR_3)_3$ and the particulate surface agent, with $R_1$, $R_2$, $R_3$ as defined herein;
- the hydrodynamic diameter of the microparticles ranges from 0.5 to 100 $\mu$m as measured by laser diffraction spectroscopy.

The microparticles are obtainable or obtained by the process described herein, in particular under section 1.

Advantageously, the microparticles are substantially free of cross-linking agent, and/or substantially free of surfactant.

As used herein, a "crosslinking agent" is a chemical reagent promoting crosslinking between the particulate surface agent and one or more products of the sol-gel reaction of the mixture comprising the tetraalkoxysilane and the trialkoxyalkylsilane. Crosslinking is a chemical reaction consisting of creating covalent chemical bonds between products that are not initially linked to each other by covalent bonds. This reaction may be initiated by heat or a pH change for instance.

As used herein, "surfactants" are typically molecular surface agents or a salt. Surfactants, such as CTAB (Cetrimonium bromide) are known in the art.

CTAB has also been described as a structuring agent. Advantageously, the microspheres are free or essentially free of CTAB.

The hydrodynamic diameter of the microparticles advantageously ranges from 2 to 80 $\mu$m, preferably from 5 $\mu$m to 50 $\mu$m, as measured by laser diffraction spectroscopy.

By definition, the hydrodynamic diameter is the diameter of a hypothetical hard sphere that diffuses with the same speed as the particle being measured. In practice, particles are solvated and can be spherical, spherical-like or non-spherical, while moving dynamically in solution. The hydrodynamic diameter is determined by calculating the Stokes-Einstein equation:

$$D_t = \frac{T k_B}{3 \pi \eta d}$$

(1)

where $T$ is the sample temperature, $\eta$ is the solvent viscosity, $k_B$ is Boltzmann's constant, and d is the sphere-equivalent hydrodynamic diameter, with the diffusion coefficient ($D_t$) calculated using varying experimental approaches. The hydrodynamic diameter is typically measured by laser diffraction spectroscopy, using for instance a litesizer 700 or scanning electron microscopy (SEM).

Advantageously, the oil core C/shell S mass ratio ranges from 40/60 to 90/10, preferably from 50/50 to 80/20.

Preferably, the loading capacity of the microparticles as determined by thermogravimetric analysis ranges from 20 % to 80%, more preferably from 50% to 80%.

Advantageously, the microparticles have a $D^{90}$ of 20 $\mu$m or less, such as from 1 $\mu$m to 20 $\mu$m. Preferably, the microparticles have a $D^{10}$ of 10 $\mu$m or less, such as from 0.5 $\mu$m to 10 $\mu$m.

As used herein, the particle size distribution is generally characterized by the "$D^{10}$" and "$D^{90}$" values . The $D^X$, where X for instance represents 10 or 50 or 90, is the $X^{th}$ percentile of the particle size distribution, in volume; that is, X% of microspheres (particles) have a size less than or equal to $D^X$ and (100-X)% of particles have a size greater than $D^X$.

*Oil core C*

The oil phase comprises a lipophilic active ingredient. The lipophilic active ingredient may be as defined above in connection with the oil phase.

Advantageously, the lipophilic active ingredient is selected from the group consisting of:

- Pest control agent, such as paraffins, in particular ($C_x$-$C_{20}$)-hydrocarbons such as ($C_x$-$C_{20}$)-*n*-alcane, in particular *n*-hexadecane, n-octadecane, *n*-eicosane;
- Essential oils;
- Perfumes;
- A vegetable oil such as an oil selected from the group consisting of neem oil, almond oil, sunflower oil, or mixtures thereof;
- Disinfectants and/or bactericides;
- Solar filters and/or UV filters, such as octylmethylcinnamate (OMC) for instance;
- Fire retardants, such as bisphenol diphenyl phosphate, resorcinol diphenyl phosphate;
- Soothing agents, such as cannabidiol;
- and mixtures thereof.

The oil core C may further comprise an oil, said oil being different from the lipophilic active ingredient. In other words, the active ingredient may or may not be solubilized in an oil. Advantageously, the oil is liquid at room temperature, that is to say the oil has a melting point of 20°C or less, preferably of 15°C or less. Preferably, the boiling point of the lipophilic active ingredient is of 70°C or more, more preferably of 80°C or more.

Advantageously, the oil is a vegetable oil such as an oil selected from the group consisting of neem oil, almond oil, sunflower oil, and mixtures thereof.

In variants where the oil core C further comprises an oil, the concentration of the lipophilic active ingredient in the oil core C in step b) preferably ranges from 0.1% to 100% v/v.

In some embodiments, the oil core C consists of the lipophilic active ingredient and optionally the oil.

*Shell S*

The shell S is the product of a sol-gel reaction of a mixture comprising or consisting of the tetraalkoxysilane, the trialkoxyalkylsilane and the particulate surface agent. The particles of the particulate surface agent are thus embedded in the shell by covalent bonds. Specifically, the particles of the particulate surface agent are covalently bonded to polysiloxanes polymeric chains, the polysiloxanes polymeric chains being the product of a sol-gel reaction of the tetraalkoxysilane, and the trialkoxyalkylsilane.

The tetraalkoxysilane is as described above. Preferably, the tetraalkoxysilane is TEOS.

The trialkoxyalkylsilane is as described above. Advantageously, the trialkoxyalkylsilane is MTES.

Preferably, the tetraalkoxysilane is TEOS and the trialkoxyalkylsilane is MTES.

The particulate surface agent is as defined above in connection with the process.

Advantageously, the particulate surface agent consists of nanoparticles, that is to say the particulate surface agent has an average particle size ranging from 1 to 100 nm, preferably from 2 to 50 nm, even more preferably from 5 to 20 nm, as measured by laser diffraction spectroscopy.

The particulate surface agent typically has a carbon content ranging from 0.5 to 50 wt%, preferably from 0.7 to 35 wt%, even more preferably from 0.8 to 10 wt%, relative to the total weight of the particulate surface agent. In some variants, the carbon content of the particulate surface agent ranges from 0.8 to 5 wt%, relative to the total weight of the particulate surface agent. In some variants, the carbon content of the particulate surface agent ranges from 12 to 40 wt%, relative to the total weight of the particulate surface agent.

## 3. Dispersion

The invention also relates to a dispersion comprising an aqueous phase and the microparticles.

In some embodiments, the dispersion may be the dispersion obtained or obtainable at the end of step d) or d3) of the process.

Alternatively, the dispersion is obtained by suspending the microparticles in an aqueous phase. Preferably, the aqueous phase has a pH ranging from 3 to 5.

In some embodiments, the dispersion further comprises an additive, in particular selected from the group consisting of thickening agents and mixtures thereof.

Examples of thickening agents are cellulose ethers, natural gums (xanthan, wellan, diutan), and polysaccharides such as starch or alginate, and combinations or derivatives thereof. Examples of cellulose ethers are methyl cellulose, methylhydroxyethyl cellulose, hydroxypropylmethyl cellulose or mixture thereof. A preferred thickening agent is alginate.

## 4. Uses

The invention also relates to a use of the microparticles or of the dispersion in a cosmetic composition, or as finishing treatment for a surface, such as a textile surface.

In cosmetic compositions, the microparticles or dispersion of the invention is useful for controlled release of cosmetic active ingredients. In particular, when formulated in a gel or cream for topical application, the active ingredient is not release within the formulation, it is only release upon applying by the user, usually on the skin.

When used for coating a surface such as a textile surface, the microparticles may be used as a surface finishing treatment such as full-bath line, coating or padding. They may also be grafted (covalently or not) to the surface. The surface treated with the microparticles or the dispersion may thus deliver the lipophilic active ingredient continuously over time, or the treatment may modify the physicochemical properties of the surface, such as porosity, air permeability, touch ...

**DRAWINGS**

Figure 1: Optical microscopy image (magnification X50) of Neem oil microcapsules. A: in water, emulsion; B: in ethanol, emulsion. C: in water, end of process; D: in ethanol, end of process

Figure 2: Optical microscopy image (magnification X50) of N-hexadecane-3 in water at 70°C, 2 hours, pH 5.

Figure 3: Optical microscopy image (magnification X50) of N-hexadecane 6 microcapsules. A: in water 70°C, 3h00, pH5; B: in ethanol 70°C, 3h00, pH5; C: in ethanol 70°C, T0, pH5.

Figure 4: Optical microscopy image (magnification X50) of N-hexadecane 8 microcapsules. A: in water 70°C 4h00 pH5; B: in ethanol 70°C 4h00 pH5; C: in ethanol 70°C T0 pH5.

Figure 5: Optical microscopy image (magnification X50) of OMC-1 microcapsules. A: in water, emulsion; B: in ethanol, emulsion; C: microcapsules in water, end of the process; D: in ethanol, end of the process.

Figure 6: Optical microscopy image (magnification X50) of OMC-2 emulsion. A: beginning of emulsion; B: after several hours of high-sheer stirring.

Figure 7: Optical microscopy observation (magnification X50) of OMC-2 microcapsules. A: in water, end of synthesis; B: microcapsules in ethanol, end of synthesis.

Figure 8: Optical microscopy image (magnification X50) of OMC-5 microcapsules. A: in water, end of synthesis; B: microcapsules in ethanol, end of synthesis.

Figure 9: Optical microscopy image (magnification X50) of OMC-6 microcapsules. A: in water, end of synthesis; B: microcapsules in ethanol, end of synthesis.

Figure 10: Optical microscopy image (magnification X50) of n-hexadecane-11 microcapsules. A: in water, end of synthesis; B: microcapsules in ethanol, end of synthesis.

Figure 11: SEM (scanning electron microscope) image of microcapsules obtained using $TiO_2$, $Al_2O_3$ and SiC as particulate surface agent.

Figure 12: Optical microscopy image (magnification X50) of Sweet almond oil/ rose of Chili oil microcapsules in water, end of synthesis.

**METHODS**

*Measurement of the average particle size by optical microscopy*

The microcapsules are monitored at various stages of the process, in water or ethanol.

A sample is collected, diluted into each solvent (water or ethanol) and observed by optical microscopy (50-fold magnification). Pictures provide information about the quality of microcapsules: their shape (spherical or not), their diameter, their integrity (broken or not), their aggregation.

Dilution in ethanol allows to investigate the robustness of microcapsules. Indeed, when particles have a broad diameter in ethanol, it shows a leak of the capsule core, meaning a weak or partially formed membrane.

Statistical analysis is used allows to determine the particle size distribution, in particular the $D^{90}$ and the $D^{10}$.

*Yield*

The chemical yield of the sol-gel reaction is calculated based on a sampling. In the following, the weight of the particulate surface agent is considered negligible.

At various stages of the process, a sample is transferred into a crucible and weighed. The crucible is left to dry in an oven at 40°C overnight. Once dried, the crucible is once again weighed, and the yield is calculated as follows:

$$Yield = \frac{w_{total} \times w_{dried\ sample}}{w_{sample} \times (w\ Hydrolyzed\ polymers + w\ Core\ material)} * 100$$

with

$w_{total}$ is the total weight of the dispersion,

$w\ dried\ sample$ is the weight of dried sample (*i.e.* powder recovered after drying),

$w\ Sample$ is the weight of raw sample (i.e. before drying)

$w\ Core\ material$ is the weight of the lipophilic active ingredient, solubilized in the oil where appropriate,

$w\ Hydrolyzed\ polymers$ is the total weight of polymers after hydrolysis, and is defined as:

$$w Hydrolyzed\ polymers = \frac{M_{hydrolyzed\ Si(OR1)4}}{M_{Si(OR1)4}} \left( w_{Si(OR1)4} \right) + \frac{M_{hydrolyzed\ R2Si(OR3)3}}{M_{R2Si(OR3)3}} \left( w_{R2Si(OR3)3} \right)$$

with $M_{hydrolyzed}$ $Si(OR_1)_4$ being the molar mass of the hydrolyzed tetraalkoxysilane, that is to say the molar mass of the $SiO_4$ residue,

$M_{Si(OR1)4}$ being the molar mass of the tetraalkoxysilane,

$M_{hydrolyzed\ R2Si(OR3)3}$ being the molar mass of the hydrolyzed trialkoxyalkylsilane, that is to say the molar mass of the $R_2SiO_3$ residue,

$M_{R2Si(OR3)3}$ being the molar mass of the trialkoxyalkylsilane,

$w_{Si(OR1)4}$ being the weight of the tetraalkoxysilane,
$w_{R2Si(OR3)3}$ being the weight of the trialkoxyalkylsilane.

For instance, for TEOS and MTES: *wHydrolyzed polymers* = $(0.288 \times w_{TEOS}) + (0.376 \times w_{MTES})$, because

$$\frac{M_{hydrolyzed\ TEOS}}{M_{TEOS}} = \frac{60}{208.3} = 0.288 \ \text{and} \ \frac{M_{hydrolyzed\ MTES}}{M_{MTES}} = \frac{67}{178.3} = 0.376 \ .$$

*Loading capacity*

The loading capacity is the mass ratio of the lipophilic active ingredient/microparticles, expressed as a % (m/m or w/w). The loading capacity may be determined by Thermo Gravimetric Analysis (TGA). For each analysis, the following parameters are used: from 25°C to 700°C, with a heating rate of 10°C per minute. Previous experiments showed that the polymeric membrane of microcapsules is stable at high temperatures, and not degraded in the temperature range used for TGA. Thus, at 700°C, the residual weight percentage determined by TGA is attributable to the shell only. The difference between the minimal residual weight (at the end of TGA) and the maximum residual weight (at 25°C, at the beginning of TGA) corresponds to the percentage of core material or loading capacity.

The theoretical loading capacity is calculated as follows:

$$Loading\ capacity\ Theoretical = \frac{w_{oil\ core}}{(w_{shell} + w_{oil\ core})} * 100$$

with $w_{oil\ core}$ representing the weight of the oil core, and $w_{shell}$ represents the weight of the shell. A good approximation of the $w_{oil\ core}$ is the addition of the weight of lipophilic active ingredient and oil where appropriate, while a good approximation of $w_{shell}$ is the $w_{hydrolyzed\ polymer}$ defined above.

### EXAMPLES

The following examples are given for illustrative purposes only and should not be understood as limiting the invention in any way.

### *Materials and Methods*

Reagents:

- Deionized water
- Acetic acid
- Particulate surface agent: Aerosil® R816 (available from EVONIK)
- Triethoxymethylsilane (MTES, CAS 2031-67-6)
- Tetraethyl orthosilicate (TEOS, CAS 78-10-4)
- Lipophilic Active ingredient: Neem oil (CAS 8002-65-1), sweet almond oil (CAS 8007-69-0), n-hexadecane (CAS 844-76-3), n-octadecane (CAS 593-45-3), n-eicosane (CAS 112-95-8) cannabidiol (CAS 13956-29-1), Octylmethoxycinnamate (CAS 5466-77-3)
- Ammonium hydroxide

Material:

- Ultrasonic homogenizer UW 2070 with probe microtip MS73 and HF generator GM 2070 (Sonopuls Bandelin)
- Dispersing machine: T25 digital U LTRA-TU RRAXO with axis 25 mm S25N-25 F (IKA®)
- RW 20 digital overhead stirrer with a 3-bladed stirrer (IKA®)
- Polystat 37 circulating bath
- Double-jacketed reactor (1L)

### General Procedure - Process of the invention

*Suspending step*

The particulate surface agent is added to a solution of acetic acid in water at a target pH at room temperature.

In a first beaker, deionized water is poured, and the pH is adjusted to the target pH by addition of acetic acid. Then, the particulate surface agent (Aerosil R816) is added. The solution 1 is homogenized with a magnetic stirrer, before being homogenized with ultrasounds at 70% for 10 min.

*Mixing step*

In a second beaker, MTES, TEOS and the lipophilic active ingredient optionally solubilized in oil are homogenized with a magnetic stirrer, at room temperature to obtain solution 2.
After the suspension has been homogenized in an ultrasonic bath, the oil phase (solution 2) is added dropwise to the solution 1 using a syringe pump, under stirring in the dispersing machine. The speed is increased to a target stirring speed of 8000 round/minutes.

*Emulsifying step*

The obtained biphasic mixture is emulsified at room temperature.
The emulsion is left to stand overnight before being transferred into a gelling double-jacketed reactor equipped with a 3-bladed stirrer.

*Gelling step*

The first step is the hydrolysis of the precursors (namely the mixture of TEOS and MTES) : The 3-bladed stirrer is placed into the reactor, and is maintained by the RW 20 digital overhead stirrer. The stirring speed is progressively increased. The reactor is heated by the circulating bath and the solution is left stirring at this temperature.
To induce polymerization, the pH of the aqueous phase is increased by dropwise addition of ammonium hydroxide. Temperature is increased to a target temperature at a rate of 1°C per minute, under stirring.

*Cooling step*

At the end of the gelling step, the suspension is left into the reactor to cool down preferably to room temperature, before being discarded into a plastic cup with a lid for storage.

*Optional Isolating step*

The suspension is filtered to isolate the microparticles, which are then placed in an oven at 35°C overnight for drying.

**Example 1 - Neem oil as lipophilic active ingredient**

**1.1 Preparation of the microparticles with neem oil core**

The microparticles are obtained following the general procedure above.
Neem oil is used as the lipophilic active ingredient. No other oil is used.
Degradation of Neem oil occurs at approximately 350°C in thermogravimetric analysis

*Suspending step*

Target pH = 3.
300mL of deionized water are used and pH is adjusted to 3 with acetic acid. 1g of particulate surface agent (Aerosil R816) is added (Solution 1). The solution is homogenized with a magnetic stirrer for approximately 10min, before being homogenized with ultrasounds for 10 min.

*Mixing step*

The mixing step of the general procedure is followed, using 13.5g of MTES, 6.5g of TEOS and 20g of the neem oil (Solution 2).
After the suspension has been homogenized in an ultrasonic bath, the oil phase (solution 2) is added dropwise to the solution 1 using a syringe pump, under stirring in the dispersing machine. The speed is increased to a target stirring at 8000 round/minutes.

*Emulsifying step*

The obtained biphasic mixture is emulsified for approximately 10 minutes at room temperature, at a stirring speed of 8000 rpm.

*Gelling step*

During the hydrolysis step, the stirring speed is progressively increased to 600 rpm. The reactor is heated by the circulating bath at 40°C (increase of 1°C/min). The solution is left to stir at 40°C for 1h30 at pH 3.

For the polymerization, the pH of the aqueous phase is then increased to 5. Temperature is increased to 70°C at a rate of 1°C per minute, and stirring is left for 5 hours.

*Isolating step*

The microparticles are obtained as a white fine powder. Yield = 96%.

## 1.2. Characterization

*Particle size*

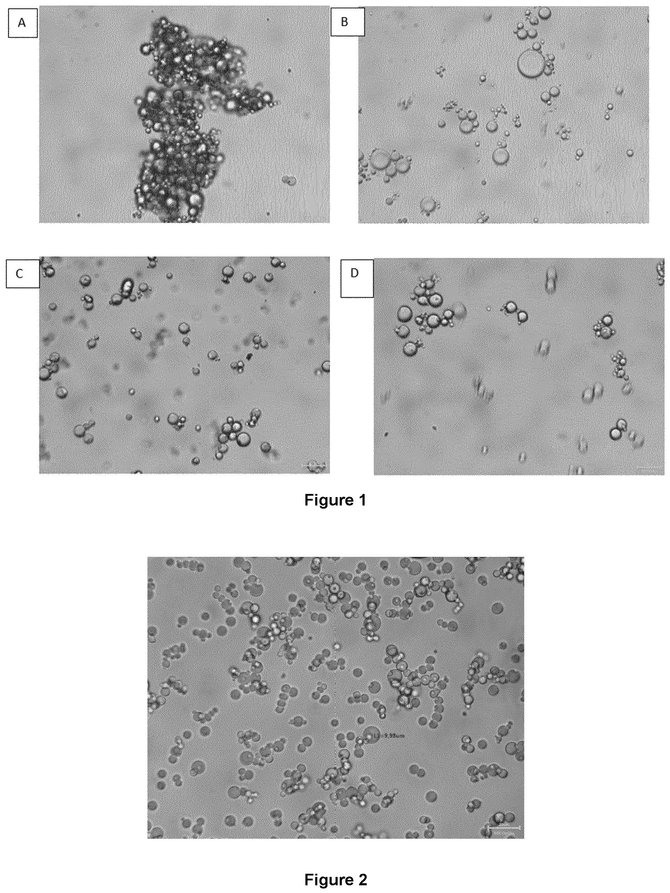

The microparticles are essentially spherical and are stable in water and ethanol under optical microscopy observation in water or ethanol (magnification x50). In both solvents, they have a similar average particle size, showing the shell's stability. Their diameter did not change during the process (see **Figure 1**).

*Loading capacity*

Based on the initial quantities of lipophilic active ingredient, tetraalkoxysilane, the trialkoxyalkylsilane, the theoretical loading capacity is of 74%.

$$Loading\ capacity\ Theoretical\ = \frac{20}{(7\ +\ 20\ )} * 100 = 74,07\%$$

According to thermogravimetric analyses, the wt% of oil core in the microparticles is of around 74%, the remaining 26% corresponding to the shell. These percentages correspond to the theoretical loading capacity, suggesting that all neem oil is encapsulated.

*Dry weight*

The dry weight of the microparticles is of around 10%.

*Encapsulation efficiency*

Lipophilic Active Ingredient (LAI) encapsulation efficiency is the weight percentage of initial neem oil effectively encapsulated. It may be calculated as follows:

$$Encapsulation\ Efficiency_{LAI}\ = \frac{\frac{w_{dried\ sample}}{w_{sample}} \times w_{total} \times \%_{LAI\ in\ \mu capsules}}{w_{Initial\ LAI}} * 100$$

With $w_{total}$ = total weight of the dispersion;
*w dried sample* = the dry weight of the sample;
*w Sample* = weight of sample (i.e. before drying);
$\%_{LAI\ in\ \mu capsules}$ = loading capacity of the microparticles, determined by TGA;
$w_{Initial\ LAI}$ = the total mass of LAI used in the process.

In the present example where LAI is Neem oil, the encapsulation efficiency is of 95.7%.

### 1.3. Diffusion assay

Diffusion of the oil core across the shell of the microcapsules is a relevant parameter to determine the porosity of the microcapsules.

To adequately determine the release kinetics of the microcapsules, the release medium should satisfy the sink conditions, *i.e.* the medium should be able to solubilize at least 3 times the weight of the oil contained in the microcapsules at the temperature of the study.

The release medium is a 5 wt% aqueous solution of Sodium Dodecyl Sulfate ("SDS") (demineralized water).

*Procedure*

The release of neem oil is studied by TGA method. Specifically, the microcapsules are added to a release medium and left under constant stirring and a given temperature for as long as possible. Samples are taken at various times, rinsed and oven-dried. The resulting powder is analyzed by TGA, to determine the remaining neem oil percentage in the microcapsules.

*Release study*

2g of microcapsules are added into 60g of release medium. The mixture is put under constant low stirring at 200 rpm, and in a water bath at 25°C. Samples are taken after 1h, 2h, 4h, 6h, 24h, 48h, 72h and 100h, rinsed several times with deionized water and oven-dried overnight at 35°C.

According to TGA profiles, the sampled microcapsules after 2 to 100 hours contain 74% of neem oil. All profiles superimpose perfectly, suggesting that the oil core is not released for at least for 100 hours.

### 1.4. scale up

The first scale-up experiment was done with the same material, the quantities of reagents were all doubled. The process produced very satisfying results: a 95% yield and a loading capacity of 73% Neem Oil and 27% of membrane. The encapsulation efficiency is approximately equal to 94%.

Then, quantities have been multiplied by 5. Here, the protocol had to be slightly modified, the reactor was changed to a 2L double-jacketed reactor. Stirring times were increased, but the reaction steps were not changed. Microcapsules are composed of 73% of neem oil and 27% of membrane, the chemical yield at the end of synthesis is equal to 97%.

It is also possible to increase dry extract, the total mass of microcapsules recovered after one synthesis, to 30%, while also increasing the total volume of synthesis. For this experiment, the synthesis yield is lower than other syntheses, but the loading capacity is maximal. In conclusion, increasing the initial quantities of reagents allows the increase of produced microcapsules, with very satisfying results. Indeed, both chemical yield and loading capacity are equivalent, regardless of the initial quantities of precursors, neem oil, nanoparticles and water. Encapsulation efficiency is similar for all syntheses, and is very satisfying: almost all Neem oil is encapsulated.

### Example 2 - N-hexadecane as lipophilic active ingredient

The following experiments aim to validate a protocol showing it is possible to increase the level of dry extract for a microcapsules syntheses containing n-hexadecane.

### 2.1. Preliminary Experiments : N-hexadecane 1 and 2

N-hexadecane oil is used as the lipophilic active ingredient. No other oil is used. Degradation of N-hexadecane oil occurs at approximately 240°C in thermogravimetric analysis

The microparticles are obtained following the general procedure above.

*Suspending step*

Target pH = 3.

300mL of deionized water are used. 1g of particulate surface agent (Aerosil R816) is added (Solution 1).

*Mixing step*

The mixing step of the general procedure is followed, using 13.5g of MTES, 6.5g of TEOS and 20g of n-hexadecane (Solution 2).

After the suspension has been homogenized in an ultrasonic bath, the oil phase (solution 2) is added dropwise to the solution 1 using a syringe pump, under stirring in the dispersing machine. The speed is increased to a target stirring of 8000 round/minutes.

*Emulsifying step*

The obtained biphasic mixture is emulsified for approximately 20 minutes at room temperature, at a stirring speed of 8000 rpm. The emulsion is stable over at least 24h.

*Gelling step*

During the hydrolysis step, the stirring speed is progressively increased to 600 rpm. The reactor is heated by the circulating bath at 40°C (increase of 1°C/min). The solution is left to stir at 40°C for 1h30.

For the polymerization, the pH of the aqueous phase is then increased to 10 with ammoniac hydroxide. Temperature is increased to 70°C at a rate of 1°C per minute, and stirring is left for 5 hours.

The results being satisfactory, the same protocol was used for one day, reducing the polymerization time to 2 hours. The results are also good, with a yield of 92%, and an n-hexadecane percentage within 73%, determined by ATG. However, polymerization at basic pH (pH approximately equal to 10, determined by pH paper) seems to lead to an aggregation of microparticles for certain active ingredients. A synthesis with polymerization at pH 5 is therefore chosen for the remainder of the experiments, in order to make the protocol feasible for different active ingredients.

## 2.2. N-hexadecane-3 (variation of final pH) (Figure 2)

N-hexadecane-3 synthesis is carried out according to protocol N-hexadecane 1, with the different steps consecutively (the emulsion was not let at rest overnight) and at a final pH equal to 5 (instead of 10) for the gelling step. The microparticles are obtained in 98% yield and the loading capacity if of 74%.

In addition, the images obtained by optical microscopy at the end of the synthesis in water show spherical, non-aggregated microcapsules of a few micrometers in diameter **(figure 2).** Thus, polymerization at pH=5 will be used during dry extract increase experiments.

## 2.3. Doubling of dry extract / N-hexadecane-6

*2.3.1. Synthesis*

A first synthesis over 2 days (emulsion left to rest overnight before gelling step) accompanied by a rise in pH to 5 was carried out.

In order to double the dry extract, the weight of active ingredients, precursors and particulate surface agent (Aerosil R816) have been doubled, only the quantity of water is unchanged (300mL). The quantities of products introduced are summarized in table 1. The homogenization time of the particulate surface agent aqueous solution is increased by 5 min, for a total ultrasonic homogenization time of 15 min.

*Table 1 : Quantities of product for the synthesis N-hexadecane 6*

|               | Product       | Quantity (g) |
| ------------- | ------------- | ------------ |
| **Precursor** | TEOS          | 13           |
|               | MTES          | 27           |
| **Nanoparticules** | Aérosil R816 | 2       |
| **Actif**     | N-hexadecane  | 40           |

*2.3.2. Characterization*

*Optical Microscopy*

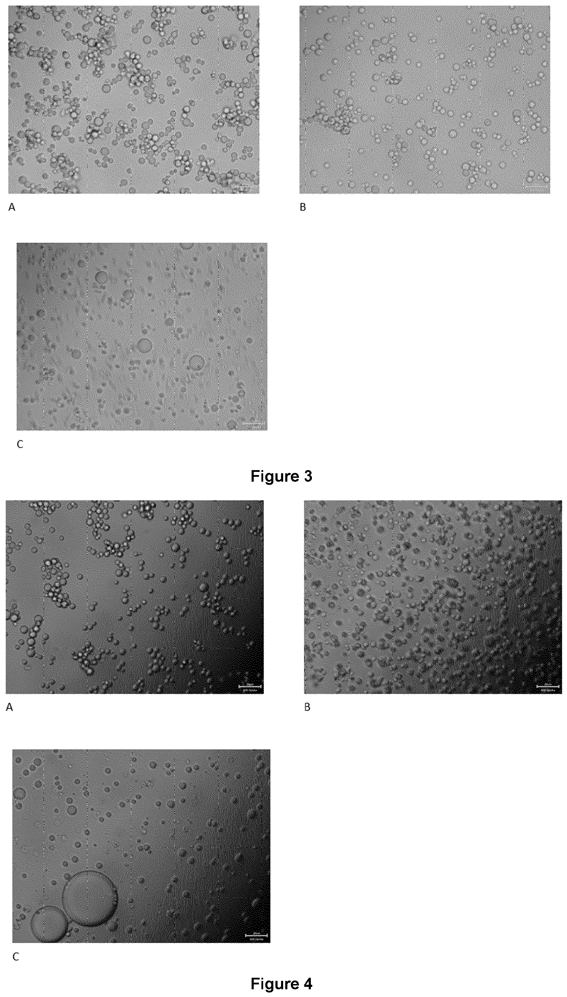

The microscopic images in the water at the end of the synthesis show spherical particles, a few micrometers in diameter and not aggregated **Figure 3A).** In ethanol, at the end of synthesis, the microcapsules are of the same size as in water **(Figure 3B),** indicating that the membrane is completely formed, contrary to the observation at 70°C T0 pH5 for example, which presents leaks **(Figure 3C).**

*Yield*

At the end of the synthesis, the yield is over 90%. Finally, the drying method with filter paper seems to be the most appropriate, taking into account numerous experiments carried out with other active ingredients. By this method, the synthesis yield is calculated at 93%.

*Table 2: Yield for N-hexadecane-6*

|  | Yield (%) |
|---|---|
| **40°C - pH3** | 79 |
| **40°C-pH5** | 80 |
| **70°C T0** | 74 |
| **70°C-1H00** | 85 |
| **70°C-2H00** | 82 |
| **70°C-3H00** | 84 |
| **After Filtration** | 93 |

*ATG*

By ATG, it was determined that, the percentage of n-hexadecane contained in the microcapsules is 74% and 26% of membrane at the end of the reaction. It is the percent expected at the end of reaction.

*2.3.3. Conclusion*

The results obtained for the N-hexadecane-6 manipulations allow us to conclude that doubling the quantities of solid without changing the quantity of liquid (here, distilled water) is possible, with good results. In comparison, with the N-hexadecane-3 experiment (dry extract at 10%), the yield is satisfactory, the active ingredient/membrane proportion is identical and equal to the theoretical result taking into account the quantities used. Furthermore, the microscopic images are similar between all the experiments, indicating on the one hand the stability of the microcapsules, and on the other hand the reproducibility of the size of the microcapsules. Indeed, by doubling the quantity of active ingredient, precursors and particulate surface agent (Aerosil R816), the size of the microcapsules does not vary.

Following these results, dry extract tripling experiments were carried out.

**2.4. tripling of dry extract -N-hexadecane-8**

*2.4.1. Synthesis*

In order to verify the possibility of tripling the dry extract, the basic protocol used for the microcapsule syntheses was initially followed. The N-hexadecane-8 manipulation carries out over 2 days, with a modification of the pH to 5 before a rise in temperature to 70°C.

The quantities of precursors, particulate surface agent (Aerosil R816) and active ingredients are tripled, these are summarized in table 3.

*Table 3: Products quantities for N-hexadecane 8 synthesis*

|  | Product | Quantity (g) |
|---|---|---|
| **Precursor** | TEOS | 19.5 |
|  | MTES | 40.5 |

(continued)

| | Product | Quantity (g) |
|---|---|---|
| **Nanoparticules of silice** | Aérosil R816 | 3 |
| **Active Ingredient** | N-hexadecane | 60 |

### 2.4.2. Characterization

*Optical Microscopie*

The microscopic images obtained at the end of the synthesis show spherical microcapsules, less than 10 $\mu$m in size, and poorly aggregated in water **(Figure 4A).** As shown in **Figure 4B,** the microcapsules are of similar size in ethanol at the end of synthesis, demonstrating the solidity of the membranes, in comparison to the observation carried out at 70°C at T0, presenting leaks in ethanol **(Figure 4C)**

*Yield*

The yield of the synthesis is presented in Table 4, according to the different stages, and according to the method described previously. At the end of the synthesis, after 4 hours of stirring at 70°C, the calculated yield is 70%, it is 78% cold, on D+1. By the filtration method using filter paper then drying in an oven at 35°C, the yield is 84%.

*Table 4: Yield for N-hexadecane-8 synthesis*

| | Rendement (%) |
|---|---|
| **40°C - pH3** | 71 |
| **40°C-pH5** | 68 |
| **70°C T0** | 71 |
| **70°C-1H00** | 77 |
| **70°C-2H00** | 65 |
| **70°C-3H00** | 74 |
| **70°C-4H00** | 70 |
| **A froid (J+1)** | 78 |
| **Filtration** | 84 |

*TGA*

The percentage of n-hexadecane within the microcapsules is high: 69% at the end of synthesis, 74% cold on D+1 and 75% cold by the filtration method on paper. The last two results correspond to the maximum percentage of encapsulated asset, according to the theoretical calculation. Thus, tripling the quantities of solids makes it possible to obtain very satisfactory results comparable to the results of previous syntheses.

### 2.4.3. Conclusion

According to the results of the N-hexadecane-8 manipulations, the tripling of the dry extract is possible with very good results, whether in terms of yield, higher than 80%, or in terms of the percentage of active ingredients contained in the microcapsules: at the end synthesis, it is higher than 70% and is close to the theoretical encapsulation rate. The microscopic images make it possible to check the appearance of the microcapsules in the water, which are spherical and not aggregated, and of similar size to each other. At the end of the synthesis, microscopic observations in ethanol show the stability of the membrane, little leakage is observed.

It is therefore possible to multiply the dry extract obtained by three, from a predefined quantity of liquid (300mL of distilled water), by tripling the quantities of precursors and active ingredients. Modifying the quantities of solid introduced does not modify the appearance of the microcapsules, their stability or their composition. It is also possible to reduce the reaction time, in order to produce the microcapsules in a single working day.

### 2.5.Conclusion on N-hexadecane experiment

The various syntheses show that the rate of dry extract obtained at the end of the reaction can be increased, by increasing the quantities of solids used (active ingredients, precursors and silica nanoparticles) and by keeping the mass ratios (Active ingredient: precursors: particulate surface agent = 1: 1: 0.05). It is possible to conserve the size of the microcapsules, which remains less than $10\mu m$ for each of the experiments. Thus, these results show that for an initial volume of distilled water of 300mL, it is possible to use up to 60g of active ingredient, 60g of precursors and 3g of nanoparticles. For all manipulations relating to the increase in dry extract, the yields and encapsulation rates at the end of synthesis are very satisfactory. Indeed, the yields are higher than 80%, or even 90% for N-hexadecane-6 syntheses. On the other hand, the encapsulation rates are important and are equivalent to the theoretical encapsulation rates calculated previously. The yield and ATG results at the end of the synthesis are summarized in Table 5.

*Table 5: Yield and quantity of active ingredient for eah synthesis*

| Synthesis | Dry extract | Yield | Actif Ingredient |
|---|---|---|---|
| N-hexadecane-3 | 10% | 98% | 74% |
| N-hexadecane-6 | 20% | 93% | 74% |
| N-hexadecane-8 | 30% | 84% | 75% |

### Example 3 - Octylmethoxycinnamate (OMC) as lipophilic active ingredient

### 3.1. OMC-1

#### 3.1.1. Preparation of the microparticles with OMC

Basically, the process consists in a three-step protocol, with varying temperatures (below 100°C) and pH throughout the synthesis. The duration of each step is below 5h. The emulsion step corresponds to the division of oil droplets into water with a high shear mixing. Then, the hydrolysis step consists in the formation of -OH functional groups on the polymers, which allows the polymerization at the interface of droplets, creating a solid membrane: it is the condensation step

The weight ratio of core material and polymers is equal to 1. At the end of the process, the total mass of microcapsules is approximately 30g, the total mass is about 300g.

Macroscopically, the final product is divided into 2 phases, as expected: a white phase containing the microcapsules below a clear liquid phase.

After drying in the oven, a white fine powder is obtained. At the end of the process, the chemical yield is equal to 96%.

#### 3.1.2. Characterization

*Microscopy*

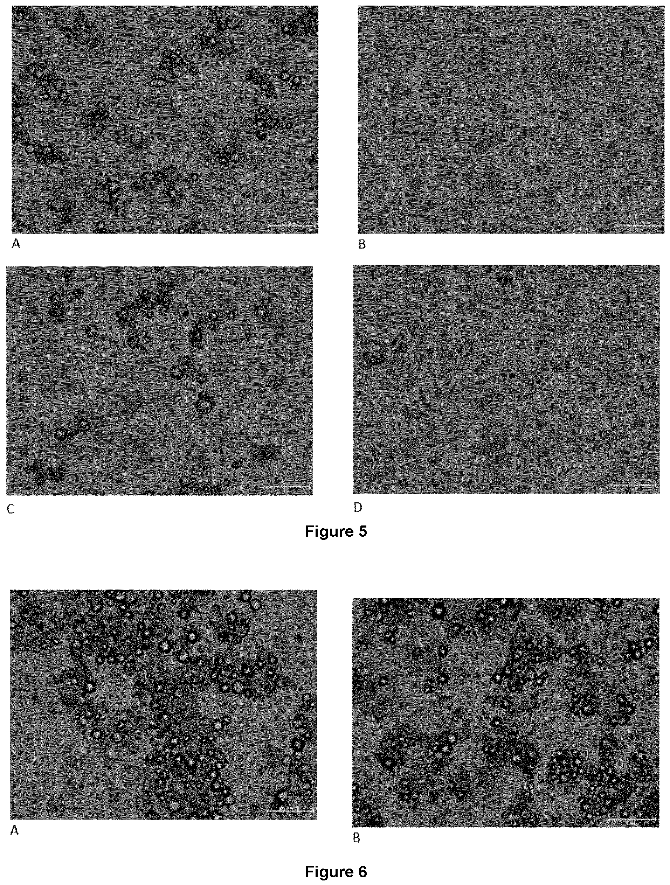

During emulsion, OMC-1 microcapsules are aggregated in water, and they seem to have a high polydispersity index. Indeed, as seen on **Figure 5A,** some microcapsules are very small, compared to other ones, it may be attributed to the viscosity of the oil. In ethanol, however, no particle is visible, suggesting that the membrane is not fully formed **(Figure 5B).**

At the end of synthesis, the microcapsules are spherical and are stable in water and ethanol (*Figures 6C and D*). In both solvents, they have a similar diameter, showing the membrane's stability. Their diameter did not change during the process.

*TGA*

According to thermogravimetric analyses, the proportion of active ingredient in the microcapsules at the end of synthesis is of 74%, the remaining 26% corresponding to the membrane. These percentages correspond to the theoretical loading capacity, suggesting that all OMC is encapsulated.

For OMC-1, the encapsulation efficiency is of 95.5%.

### 3.2. OMC-2

### 3.2.1. Preparation of the microparticles

As seen on the microscopic observations of OMC-1, microcapsules are of different sizes: the size varies between < 1μm and 6 μm. In order to decrease polydispersity, the synthesis has been reproduced by increasing the duration of the emulsion step. The process used is the same as for OCM-1

At the beginning of the emulsion step, microcapsules are polydisperse, a large proportion of particles have a broad diameter (see **Figure 6A**). As shown on **Figure 6B,** when increasing stirring time, the large particles seem to decrease slightly in size.

Then, the synthesis is continued, following the same steps as for OMC-1.

The chemical yield is calculated as 94%, and the final dispersion, when left to rest, consists in a white phase containing the microcapsules below a clear liquid phase.

### 3.2.2. Characterization

*Microscopy*

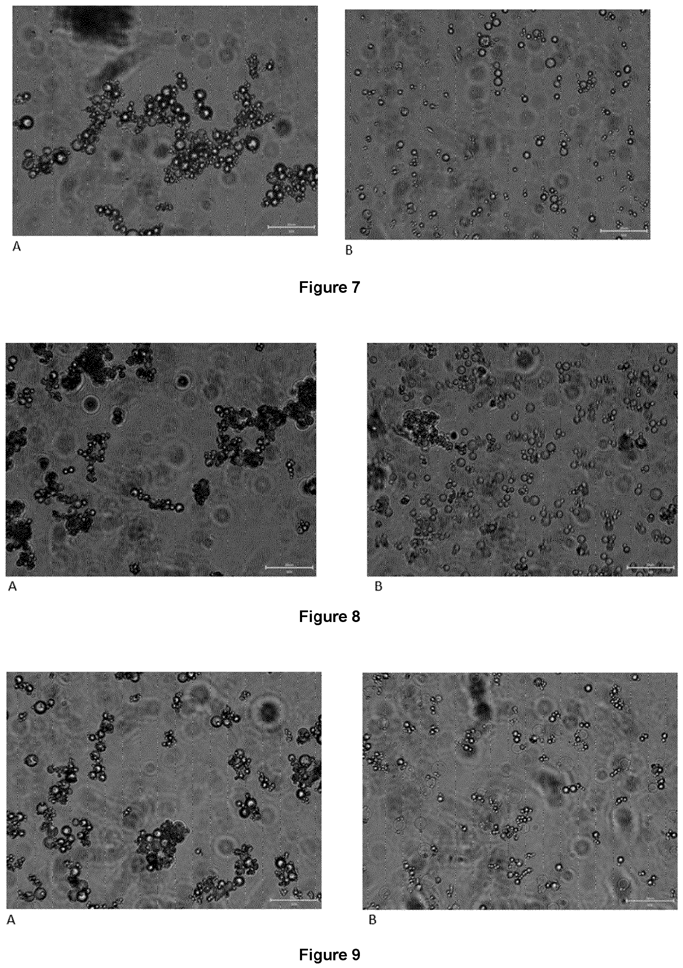

As seen on **Figures 7A and 7B,** at the end of synthesis, microcapsules are spherical, but their sizes vary in water. In ethanol, sizes are also different between capsules. They remain spherical, and no leaks are observed, suggesting the stability of the membranes.

*TGA*

According to thermogravimetric analysis capsules consist in 72% of core material (OMC) and 28% of membrane. However, as seen on the TGA curve, the degradation profile is slightly different than OMC-1, indeed, a second degradation seems to occur between 300 and 450°C; it could be because all of the precursors did not react together to form the membrane. Nevertheless, this second degradation does not modify the properties of the microcapsules, as their degradation temperature is identical to OMC-1 microcapsules (approximately 200°C), and the core proportion is high and similar to OMC-1.

Encapsulation efficiency is calculated as 91%, slightly below the one calculated for OMC-1.

### 3.3. OMC-5

### 3.3.1. preparation

In the example, the same procedure as above for OMC-1 and OMC-2 was followed, except that the quantities of non-aqueous reagents were tripled. Consequently, the duration of the stirring steps was also increased.

Macroscopically, at the end of the process, white microcapsules are at the bottom of the container, and a clear transparent aqueous phase at the top, as expected. The proportion of the microcapsules phase is increased compared to the previous synthesis, following the mass ratios of the reagents.

After drying in the oven, a white fine powder is obtained, and the chemical yield is calculated as 80%.

### 3.3.2. Characterization

*Microscopy*

Microscopic observations show that at the end of the synthesis, microcapsules are spherical in water and ethanol **(Figures 8 A and B).** In ethanol, no particle is transparent, suggesting the stability of the membrane.

*TGA*

Microcapsules are composed of 73% of OMC, and 27% of membrane. Increasing the dry extract mass proportion does not seem to impact the results obtained. In any case, the microspheres obtained with tripled proportions are similar to those obtained with a composition corresponding to a dry extract of 10%.

Encapsulation efficiency is of 79%.

### 3.4. OMC-6

The OMC-6 synthesis followed the same steps as the previous syntheses, however, the total quantities of reagents were doubled, to multiply the total volume of the synthesis by 2. Material adaptations were made.

At the end of the process, and as expected, microcapsules are white, and the container shows two phases: a liquid transparent one, above the phase containing the microcapsules.

Once dried, a white fine powder is obtained, and OMC-6 chemical yield is calculated as 94%. Microscopic observations show that at the end of the synthesis, microcapsules are spherical in water and ethanol **(Figures 9A and 9B).** In ethanol, however, some particles remain transparent, suggesting that membranes are not stable. Increasing synthesis time may compensate for this effect. It is possible that multiplying the volume of the synthesis by two necessitates adjustments in the protocol, to obtain stable particles.

OMC accounts for 73% of the microcapsules. The same results were obtained with other syntheses, doubling the total weight of the synthesis leads to very satisfying results. However, a second degradation is observed between 350 and 450°C.

Encapsulation efficiency is equal to 92%.

### 3.5 Conclusion

Encapsulation of Octyl MethoxyCinnamate (OMC) has been done by following the generic process. OMC-1, and OMC-2 syntheses show very satisfying yield, encapsulation efficiency, and loading capacity, with good repeatability. Microscopic observations show, however, that polydispersity is high. Increasing the duration of the emulsion step allows the decrease in size of the largest particles. Nevertheless, the size difference of the microcapsules does not modify their properties.

Multiplying the weight of the reagents by three, but keeping the same total volume of water allows very satisfying results, as demonstrated by OMC-5 synthesis. The total quantity of microcapsules recovered after one synthesis is then tripled.

It is also possible to double the total volume of synthesis, as suggested by the results obtained for OMC-6.

However, for some samples, TGA profiles display a second degradation step, which was not observed on the TGA profiles of raw OMC. Fourier Transform Infrared spectra of microcapsules heated to various temperatures do not show any structural modifications of the molecule. It is however possible that remaining free hydroxyl groups of the polymer network reacted with OMC at high temperatures. The spectra suggest that this reaction takes only place at the high temperatures used for the thermogravimetric analyses. Therefore, it might be an artefact associated with the measuring method.

Experiments with a viscosifying agent were shown to improve stability of microcapsules at high temperatures, as with other core materials.

### Example 4 : Thickened dispersion using alginate

The objective of example 4 is to evaluate the effect of viscosifying agents in the formulas, in order to increase thermal stability of microcapsules.

It has been shown that adding a thickening agent into a melamine-based microcapsules dispersion allows to mask the membranes' porosity, thus improving thermal stability: loss of mass occurs at higher temperatures than microcapsules without thickening agent.

For these experiments, the chosen thickening agent has been added into liquid samples of microcapsules dispersions, before being dried and left in the oven at high temperatures, which allows to evaluate the remaining quantity of active ingredient in the microcapsules by TGA.

### 4.1. Alginate solutions preparation

Sodium alginate has been chosen as the viscosifying agent, because of its availability in the laboratory and its ease of use.

Alginate necessitates a high shear stirring to disperse into water without forming clusters. It is then impossible to directly mix sodium alginate and microcapsules suspensions. Therefore, 10% alginate in de-ionized water solutions were prepared, and were left untouched for 2 days, to attain the final viscosity.

Alginate solutions were prepared as follows:

In a beaker, weight the desired de-ionized water and stir vigorously, until the formation of a vortex. Separately, weight the desired sodium alginate mass, and add it gradually but rapidly to the water. Leave it under stirring until complete dissolution of sodium alginate.

### 4.2. N-hexadecane-11 Synthesis

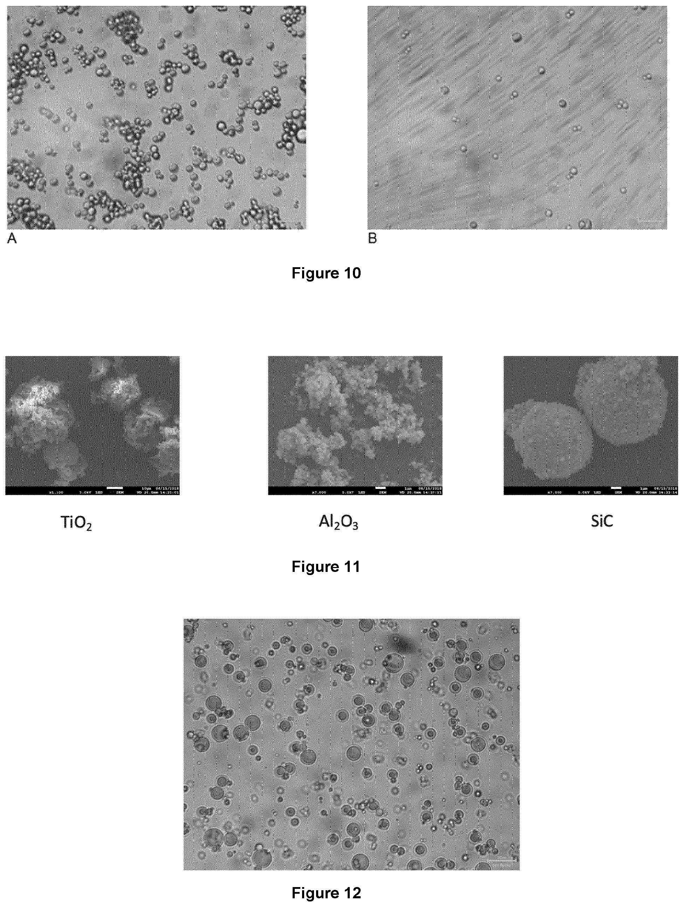

N-hexadecane-11 synthesis is carried out according to protocol N-hexadecane 3 (example 2), At the end of synthesis, in water, microcapsules are spherical and stable **(Figure 10A).** Their diameter varies between 4 and 8$\mu$m. In ethanol, microcapsules are spherical, stable, and no leaks are visible, suggesting the membrane's stability **(Figure10B).**

The chemical yield is equal to 76%, and, as determined by TGA, microcapsules contain 74% of core material (n-hexadecane), which is the maximum loading capacity according to the initial quantities of oil, polymers and nanoparticles.

Because of the very satisfying results, N-hexadecane-11 synthesis has been used for the assays with alginate.

### 4.3. Thickening agent assays

Samples of liquid N-hexadecane-11 were taken, and various quantities of sodium alginate solutions were added by magnetic stirring. Table 6 summarizes the different analyses:

*Table 6: Summary of the alginate assays*

| Weight N-hexadecane-11 | Weight Alginate solution | % Alginate |
|---|---|---|
| 10g | 3g | 5% |
| 10g | 4,5 g | 6% |
| 10g | 2g | 4% |

After the addition of the 10% sodium alginate solution into the microcapsules' dispersions, the samples are dried in the oven at 35°C. The recovered powder is then placed in the oven at 150°C for 3 hours. A sample of dried N-hexadecane-11 microcapsules, without alginate, follows this step as well, in order to compare results with and without thickening agent.

TGA profile indicates that after 3 hours at 150°C, the membrane accounts for 92% of the microcapsules, the core material thus representing only 8% of the total mass. N-hexadecane-11 microcapsules are composed by 26% of membrane and 74% of core material. Therefore, the loss of core material after 3 hours at 150°C indicates that high temperatures degrade the encapsulated n-hexadecane. It is then relevant to evaluate the n-hexadecane loss when sodium alginate is added: indeed, the thickening agent would fill the pores at the surface of the microcapsules' shell, making them more stable at high temperatures.

TGA profiles of microcapsules with alginate show a reduced n-hexadecane degradation. Indeed, whereas non-treated microcapsules only contain 8% of n-hexadecane, microcapsules treated with 4, 5 and 6% sodium alginate seem to contain 18%, 24% and 28% of n-hexadecane.

### 4.4. Conclusion

The addition of alginate into microcapsules dispersions seems to be beneficial to the microcapsules stability. Indeed, when the percentage of added thickening agent increases, the remaining percentage of n-hexadecane inside the microcapsules increases.

### Example 5: influence of the nature of the particulate surface agent

The particulate surface agent (TiO$_2$, or Al$_2$O$_3$ or SiC) is added to a solution of acetic acid in water at a target pH at room temperature.

In a beaker, deionized water is poured, and the pH is adjusted to the target pH by addition of acetic acid. Then, the particulate surface agent (TiO$_2$, or Al$_2$O$_3$ or SiC) is added. The solution 1 is homogenized with a magnetic stirrer, before being homogenized with ultrasounds at 70% for 10 min. The next steps are the same as those of the general procedure.

In all cases, the core is well contained in the particles without noticeable change in encapsulation performance.

Depending on the type of particulate surface agent (morphology and chemical nature, in particular carbon content) used to stabilize the emulsion, the final morphology of the microcapsules is different.

The use of SiC, which is hydrophobic, results in well-defined spherical particles with surface roughness (see **figure 11C).**

In contrast, the use of TiOz or Al$_2$O$_3$ leads to submicrometric or nanometric capsules with a solid tendency to agglomerate. In other words, TiOz and Al$_2$O$_3$ do not allow to obtain well-defined microparticles (see **figures 11A and B).**

### Example 6 - **Mixture of Sweet almond oill Chili rose oil as lipophilic active ingredient**

**6.1 Preparation of the microparticles with sweet almond oil core**

The microparticles are obtained following the general procedure above.

*Suspending step*

Target pH = 3.
300mL of deionized water are used. 1g of particulate surface agent (Aerosil R816) is added (Solution 1).

*Mixing step*

The mixing step of the general procedure is followed, using 13.5g of MTES, 6.5g of TEOS and 20g of Sweet almond oil/ rose of Chili oil (Solution 2).
After the suspension has been homogenized in an ultrasonic bath, the oil phase (solution 2) is added dropwise to the solution 1 using a syringe pump, under stirring in the dispersing machine. The speed is increased to a target stiring at 8000 round/minutes.

*Emulsifying step*

The obtained biphasic mixture is emulsified for approximately 20 minutes at room temperature, at a stirring speed of 8000 rpm. The emulsion is stable at least 24h.

*F figureGellinq step*

During the hydrolysis step, the stirring speed is progressively increased to 600 rpm. The reactor is heated by the circulating bath (double jacket) at 40°C (increase of 1°C/min). The solution is left to stir at 40°C for 1h30.
For the polymerization, the pH of the aqueous phase is then increased to 5 with ammoniac hydroxyde. Temperature is increased to 70°C at a rate of 1°C per minute, and stirring is continued for 2 hours.

**6.2. Characterization**

*Optical Microscopie*

The microscopic images obtained at the end of the synthesis show spherical microcapsules, less than 15 $\mu$m in size (see **Figure 12).**

*Yield*

The yield of the synthesis is 97%.

*TGA*

The percentage of Sweet almond - Chili rose oil / membrane is 73%/27% as expected.

**Example 7 - Cannabidiol (CBD) as lipophilic active ingredient**

CBD oil is hemp oil with the addition of 32% CBD.
The basic protocol was followed over 2 days, at pH5. In the water and ethanol, the particles are spherical, nevertheless, their diameter is very variable: it is probable that this is related to the viscosity of the oil. In ethanol, leaks are present throughout the synthesis process, on day D+ 1.
Also, some are slightly deformed or collapsed. After drying, the microcapsules are fine visible and spherical. The yield at the end of synthesis is 96%, the percentage of encapsulated oil is 72%. Nevertheless, on the ATG profiles, 2 successive degradations are visible: it could be a thermal degradation of components during PTA, having no effect on the composition of the microcapsules, silanes could also react with certain constituents of the oil.

**Claims**

1. Microparticles comprising an oil core C and a shell S, wherein

   • the oil core C comprises a lipophilic active ingredient;
   • the shell S is a product of a sol-gel reaction of a mixture comprising a tetraalkoxysilane of formula $Si(OR_1)_4$, a trialkoxyalkylsilane of formula $R_2Si(OR_3)_3$ and a particulate surface agent with a carbon content ranging from 0.5 to 50 wt% relative to the total weight of particulate surface agent,
   $R_1$ being independently a $(C_1\text{-}C_6)$alkyl, $R_2$ being a $(C_1\text{-}C_6)$alkyl, and $R_3$ being independently a $(C_1\text{-}C_6)$alkyl;
   • the hydrodynamic diameter of the microparticles ranges from 0.5 to 100 $\mu$m as measured by laser diffraction spectroscopy.

2. The microparticles of claim 1, wherein the microparticles are substantially free of cross-linking agent, and/or substantially free of surfactant.

3. The microparticles of claim 1 or 2, wherein the particulate surface agent is selected from the group consisting of:

   - a silica carbide or a metal carbide such as aluminum carbide
   - a hydrophobized silica;
   - a hydrophobized mixture of silica with a metal oxide, such as titanium oxide, manganese oxide, calcium oxide, calcium carbonate, zirconium oxide, or mixtures thereof;
   - or mixtures thereof.

4. The microparticles of any of claims 1 to 3, wherein the tetraalkoxysilane is tetraethoxysilane, and the trialkoxyalkylsilane is triethoxymethylsilane.

5. The microparticles of any of claims 1 to 4, wherein the oil core C further comprises an oil, said oil being different from the lipophilic active ingredient.

6. The microparticles of any of claims 1 to 5, wherein the loading capacity of the microparticles ranges from 20% to 80 % as measured by thermogravimetric analysis.

7. A process for preparing the microparticles of any of claims 1 to 6, comprising the following steps:

   a) Suspending a particulate surface agent with a carbon content ranging from 0.5 to 50 wt% relative to the total weight of particulate surface agent, in an aqueous phase Aq, preferably having a pH ranging from 3 to 10, to obtain an aqueous suspension,
   b) Mixing the aqueous suspension with an oil phase comprising a lipophilic active ingredient, a tetraalkoxysilane of formula $Si(OR_1)_4$, and a trialkoxyalkylsilane of formula $R_2Si(OR_3)_3$, with $R_1$, $R_2$ and $R_3$ as defined in claim 1, to obtain a biphasic mixture,
   c) Emulsifying the biphasic mixture to obtain an oil-in-water emulsion,
   d) Subjecting the oil-in-water emulsion to gelling conditions, so as to obtain a dispersion of microparticles comprising the oil core C encapsulated into a shell S which is the product of a sol-gel reaction of a mixture comprising the tetraalkoxysilane, the trialkoxyalkylsilane and the particulate surface agent.

8. The process of claim 7, wherein the oil-in-water emulsion has an aqueous phase having a pH ranging from 3 to 10, preferably 3 to 6, and the step d) of subjecting the emulsion to gelling conditions comprises the following sub-steps:

   d1) Adding a base to the oil-in-water emulsion so as to raise the pH of the aqueous phase of the emulsion to a range from 5 to 11, preferably from 9 to 10, thereby obtaining an emulsion with an adjusted pH,
   d2) Heating the emulsion with an adjusted pH to a temperature below the lowest ebullition temperature between the ebullition temperature of the lipophilic active ingredient, water, the trialkoxyalkylsilane and the tetraalkoxysilane, preferably ranging from 45°C to 80°C such as from 50°C to 70°C, to obtain a hot dispersion,
   d3) Cooling the hot dispersion to room temperature.

9. The process of claim 7 or 8, wherein the oil phase may further comprise an oil different from the lipophilic active ingredient.

10. The process of any of claims 7 to 9, wherein the trialkoxyalkylsilane/tetraalkoxysilane mass ratio in step b) ranges from 30/70 to 70/30, preferably from 40/60 to 60/40, even more preferably from 45/55 to 55/45.

11. The process of any of claims 7 to 10, wherein the dry weight of the biphasic mixture ranges from 5% to 50%, preferably from 10% to 40% (m/m).

12. The process of any of claims 7 to 11, wherein the oil-in-water emulsion obtained in step c) comprises oil droplets having a number-average diameter ranging from 0.5 $\mu$m to 50 $\mu$m, preferably from 50 $\mu$m to 20 $\mu$m.

13. The process of any of claims 7 to 12, wherein the process further comprises a subsequent step e) of isolating, for instance by filtration, and optionally drying the microparticles.

14. A dispersion comprising an aqueous phase and the microparticles of any of claims 1 to 6 or obtained by the process of any of claims 7 to 13.

15. Use of the microparticles of any of claims 1 to 6 or obtained by the process of any of claims 7 to 13, or the dispersion of claim 14, in a cosmetic composition, or as finishing treatment for a surface, such as a textile surface.

Figure 1

Figure 2

A

B

C

**Figure 3**

A

B

C

**Figure 4**

A

B

C

D

Figure 5

A

B

Figure 6

A

B

Figure 7

A

B

Figure 8

A

B

Figure 9

A                                                    B

Figure 10

TiO$_2$                          Al$_2$O$_3$                          SiC

Figure 11

Figure 12

**Europäisches Patentamt**
**European Patent Office**
**Office européen des brevets**

# EUROPEAN SEARCH REPORT

**Application Number**

EP 24 30 7102

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BUTSTRAEN CHLOÉ ET AL: "Sol-gel microencapsulation of oil phase with Pickering and nonionic surfactant based emuls", POWDER TECHNOLOGY, vol. 284, 3 July 2015 (2015-07-03), pages 237-244, XP029262091, ISSN: 0032-5910, DOI: 10.1016/J.POWTEC.2015.06.055 * paragraph [02.2]; figure 4 * | 1-15 | INV. A61K8/04 A61K8/11 |
| A | Salaün Fabien ET AL: "Sol-Gel Microencapsulation Based on Pickering Emulsion" In: "Science and Technology Behind Nanoemulsions", 22 August 2018 (2018-08-22), InTech, XP093277602, ISBN: 978-1-78923-571-5 pages 1-23, DOI: 10.5772/intechopen.74299, | 1-15 | |
| Y | WO 2011/124706 A1 (BASF SE [DE]; DREHER JING [DE] ET AL.) 13 October 2011 (2011-10-13) * claims 1, 12, 16; examples 1-4 * | 1-15 | **TECHNICAL FIELDS SEARCHED (IPC)** A61K A61Q |

The present search report has been drawn up for all claims

| Place of search | Date of completion of the search | Examiner |
|---|---|---|
| Munich | 16 May 2025 | Krattinger, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 30 7102

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | Publication date | Patent family member(s) | Publication date |
|---|---|---|---|
| WO 2011124706 A1 | 13-10-2011 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 30072020 A

**Non-patent literature cited in the description**

- **ZHAO et al.** *Langmuir*, 2014, vol. 30, 4253-4261
- **BUTSTRAEN et al.** *Powder Technology*, 2015, vol. 284, 237-244
- M9 Biopharmaceutics Classification System Based Biowaivers - Guidance for Industry. American FDA (Food and Drug Administration), May 2019
- *CHEMICAL ABSTRACTS*, 2031-67-6
- *CHEMICAL ABSTRACTS*, 78-10-4
- *CHEMICAL ABSTRACTS*, 8002-65-1
- *CHEMICAL ABSTRACTS*, 8007-69-0
- *CHEMICAL ABSTRACTS*, 844-76-3
- *CHEMICAL ABSTRACTS*, 593-45-3
- *CHEMICAL ABSTRACTS*, 112-95-8
- *CHEMICAL ABSTRACTS*, 13956-29-1
- *CHEMICAL ABSTRACTS*, 5466-77-3